# EUROPÄISCHE PATENTSCHRIFT

(11) **EP 2 259 752 B1**
(45) Veröffentlichungstag und Bekanntmachung des Hinweises auf die Patenterteilung: **12.11.2014**
(21) Anmeldenummer: 09722266.5
(22) Anmeldetag: 16.03.2009
(51) Int. Cl.: A61F 2/36

(54) **PROTHESENKÖRPER FÜR EINE OBERSCHENKEL-PROTHESE**
PROSTHETIC BODY FOR AN UPPER LEG PROSTHETIC
ÉLÉMENT PROTHÉTIQUE POUR UNE PROTHÈSE FÉMORALE

(30) Priorität: 17.03.2008 DE 102008014466
(43) Veröffentlichungstag der Anmeldung: 15.12.2010
(73) Patentinhaber: Copf, Franz, senior, 71638 Ludwigsburg (DE)
(72) Erfinder: Copf, Desirée, 71638 Ludwigsburg (DE); Copf, Peter, 71686 Remseck am Neckar (DE); Copf, Franz, senior, 71638 Ludwigsburg (DE)
(74) Vertreter: Borkowski, Jens
(86) Internationale Anmeldenummer: PCT/EP2009/001929
(87) Internationale Veröffentlichungsnummer: WO 2009/115281

(56) Entgegenhaltungen:
- EP-A- 0 078 888
- CH-A5- 678 807
- DE-A1- 2 331 728
- FR-A- 2 873 568
- US-A- 4 051 559
- US-A- 4 163 292
- US-A1- 2005 004 680

## Beschreibung

Die Erfindung betrifft einen Prothesenkörper für eine Oberschenkel-Prothese nach Anspruch 1. Dieser umfasst einen Schaft mit einer proximalen Tragplatte für einen Prothesenkopf, wobei an der Tragplatte von dieser herabhängend wenigstens zwei proximal beabstandete und distal aufeinander zulaufende, insbesondere im distalen Endbereich miteinander verbundene Schaftpfosten befestigt sind, insbesondere die durch Verstrebungen, wie z.B. Schrägverstrebungen verbunden sind und wobei zwischen den Schaftpfosten und den ggfs. vorgesehenen Verstrebungen Ausnehmungen und/oder frei durchgängige Bereiche gebildet sind, insbesondere in die Spongiosa einwachsen kann. Die Schaftpfosten entsprechen ihrem Verlauf nach der Anatomie des Knochens und vergrößern die Oberfläche des Prothesenschaftes unter minimalem Einsatz von Metallvolumen. Jeder der Schaftpfosten kann entlang seiner Erstreckung, insbesondere medial bzw. lateral eine Materialaussparung aufweisen und somit auch als zwei verbundene parallele Schaftpfosten verstanden werden.

Die Prothesenkörper der genannten Art sind dabei im Stand der Technik bekannt und bilden bei der beschriebenen Ausführungsform einen Prothesenkörper, der in den Knochen einwächst, d.h. bei dem die Spongiosa nach einer Ausheilzeit die Ausnehmungen und/oder frei durchgängigen Bereiche zwischen den Schaftpfosten und den Verstrebungen vollständig ausfüllt, so dass ein derartiger Prothesenkörper fest im Knochen verankert ist, ohne dass es einer Einzementierung bedarf.

Prothesenkörper werden z.B. in folgenden Dokumenten beschrieben: DE 23 32 728 A1,FR welche die Basis für den Obergriff von Anspruch 1 bildet, sowie in 2 873 568 A, US 4 163 292 A, US 4 051 559 A, CH 678 807 A5, US 2005/004680 A1 und EP-A-0 078 888.

Die bei dieser Patentbeschreibung verwendeten Richtungsangaben beziehen sich hier auf die Lage des Prothesenkörpers nach Einbringung in den Oberschenkelknochen bei einem aufrecht stehenden Menschen.

Bei einem hier beschriebenen Prothesenkörper kann es weiterhin ergänzend vorgesehen sein, dass die Schaftpfosten entlang ihrer Längserstreckung zusätzlich beabstandete Verankerungsbunde, insbesondere ringförmige Verankerungsbunde aufweisen. Solche Verankerungsbunde können ausgebildet sein als radiale Verdickungen, die der Oberflächen-Vergrößerung dienen, was auch durch andere geometrische Formen erreicht werden kann. Z.B. können die Verankerungsbunde auch als Rillen ausgebildet sein. Zusätzlich zu den Schaftpfosten können auch die ggfs. vorgesehenen Verstrebungen derartige Verankerungsbunde haben.

Prothesenkörper dieser Art werden üblicherweise aus einer Titanlegierung oder auch aus Chrom-Kobalt-Molybdän-Legierungen oder anderen geeigneten Materialien gefertigt und können darüber hinaus zwecks besserer Verbindung mit der Knochenmasse eine mikroporös aufgeraute Oberfläche aufweisen, die z.B. durch Strahlen mit Glasperlen / Partikeln oder auch durch Ätzen erzeugt werden kann.

Durch die beschriebene Konstruktion, bei der die Schaftpfosten im oberen Bereich, in welchem sie mit der Tragplatte verbunden sind, beabstandet sind und nach unten hin aufeinander zulaufen, insbesondere am distalen Ende miteinander verbunden sind, wobei die Pfosten einen gekrümmten Verlauf einnehmen können, wird eine Formgestaltung erzielt, die dem inneren freien Bereich des proximalen Endes eines Oberschenkelknochens nach Ausraspeln der spongiösen Knochenstruktur entspricht. So passt sich eine derartige Prothese optimal an das Innere des Knochens an.

Im Stand der Technik ist es bei dieser Art von Prothesen bekannt, dass die Tragplatte allseitig über den Schaft, bzw. den Querschnitt des Schaftes herüberragt, der unmittelbar unterhalb des Verbindungsbereiches zwischen Tragplatte und Schaftpfosten gegeben ist. Somit weist eine bekannte Tragplatte bisheriger Prothesenkörper gegenüber dem Schaft eines solchen Prothesenkörpers einen allseitigen Kragen auf. In einem derartigen Kragen wurden bislang die Vorteile erkannt, dass sich dieser rund um auf dem Corticalisknochen des Femurschaftes abstützen kann.

Zwischenzeitlich wurde herausgefunden, dass aufgrund unzureichender Erkenntnis über die physiologischen Eigenschaften der Kraftübertragung im Oberschenkelknochen die Tragplatte der bisher im Stand der Technik bekannten Prothesenkörper zu groß angelegt wurde. Oft musste ein derartiger Prothesenkörper von lateral in das Spongiosavolumen eingebracht werden, wobei man hierzu eine Osteotomie des Trochanter major durchführte. Dieser musste sodann an den Prothesenkörper mittels einer Drahtschlinge fixiert werden, was zur Stabilisierung der Zugkräfte der Muskulatur diente.

In der Zwischenzeit wurde herausgefunden, dass eine derartige proximale Resektion des proximalen Femurs nicht zwingend notwendig ist.

Aufgabe der Erfindung ist es daher, einen Prothesenkörper der eingangs genannten Art bereitzustellen, ferner wird als Beispiel ein dafür verwendbares Raspelinstrument gezeigt, mit denen gemeinsam zum einen mit geringerem Materialeinsatz beim Prothesenkörper und zum anderen mit geringeren Eingriffen am Oberschenkelknochen eine festsitzende und allen Belastungen des Alltags standhaltende Prothese erzielt werden kann.

Die Erfindung wird dadurch gelöst, dass bei einem Prothesenkörper der eingangs genannten gattungsgemäßen Art zumindest der untere Verbindungsbereich der Tragplatte, an dem die Schaftpfosten befestigt sind, horizontal angeordnet ist, die Tragplatte nach medial über den unmittelbar unterhalb des Verbindungsbereiches gegebenen Schaft bzw. dessen Querschnitt übersteht und die Tragplatte ventral und dorsal ohne Überstand in den Schaft übergeht.

Ein derartiger erfindungsgemäßer Prothesenkörper stützt sich somit im Wesentlichen nur noch medial über den dort vorspringenden Bereich der Tragplatte an dem Corticalisknochen des Femurs ab, nämlich hier insbesondere an der Stelle, wo der Corticalisbereich besonders stark und fest ausgebildet ist, sowie mit seiner stark vergrößerten Oberfläche auf den im Spongiosarestvolumen verbliebenen Spongiosatrabekeln. Hingegen ventral und dorsal weist die Tragplatte keinen bzw. keinen wesentlichen Überstand gegenüber dem Querschnitt des Schaftes unmittelbar unterhalb des Verbindungsbereiches auf, so dass die Tragplatte mit ihren ventralen und dorsalen Seitenbereichen im Wesentlichen nur noch medial im Femur angeordnet ist, d.h. in diesen Bereichen nicht auf dem Corticalisknochen aufliegt, wie dies bei den Ausführungen gemäß Stand der Technik bislang der Fall war. So kann bei einem erfindungsgemäßen Prothesenkörper eine ca. 4-6ml größere Resektion ausgeführt werden, was das Einwachsen des Prothesenkörpers nicht negativ beeinflusst, dadurch aber eine kleinere Tragplatte ausreicht, da distalwärts reseziert wird, und somit Material am Prothesenkörper eingespart werden kann.

Bei der genannten erfindungsgemäßen Ausführung, gemäß der die Tragplatte ventral und dorsal ohne Überstand in den Schaft übergeht, wird unter dem Merkmal, dass kein Überstand vorhanden ist, nicht nur ein mathematisch exaktes Fluchten zwischen Schaft und Tragplatte verstanden, sondern auch geringe Überstände, die jedoch derart gering sind, dass sich kein Kragen ausbildet, mit dem ein Abstützen auf dem Corticalisknochen des Femurs stattfindet.

Unter Tragplatten ohne Überstand werden demnach solche verstanden, die mit ihren ventral und dorsal angeordneten Seitenflächen medial im Femur angeordnet sind. Dabei können Überstände zum Schaft im Bereich von 0 - 10 %, bevorzugt 0 - 5 % bezogen auf die Dicke des Schaftes zwischen den ventralen und dorsalen Seitenflächen der Tragplatte gegeben sein. Tragplatten mit derart geringen Überständen werden als Tragplatten verstanden, die erfindungsgemäß ohne Überstand ausgebildet sind im Vergleich zu denjenigen Tragplatten wie sie aus dem Stand der Technik bekannt sind.

Ansonsten kann ein Prothesenkörper in üblicher Art und Weise, wie es bei den Prothesenkörpern dieser gattungsgemäßen Art bekannt ist, in den Femur implantiert werden, wobei das Metallfreie Volumen des Prothesenschaftes entweder mit auto oder künstlichem Spongiosatransplantat aufgefüllt werden soll.

In einer besonders bevorzugten Weiterbildung kann es vorgesehen sein, dass der Prothesenkörper lateral im Bereich der Tragplatte, bzw. die Tragplatte selbst lateral derart endet, dass der Prothesenkörper bzw. die Tragplatte nach einer Insertion in den Femur zumindest überwiegend anlagefrei zum Corticalisknochen des Trochanter major ist.

So ist es aus dem Stand der Technik bislang bekannt, dass Prothesenkörper der eingangs genannten Art lateral an der Tragplatte einen lateral und nach oben weisenden Vorsprung insbesondere gebogener Art aufweisen, mit dem sich der bekannte Prothesenkörper an der Corticalis des Trochanter major abstützt.

Es wurde durch umfangreiche Untersuchungen herausgefunden, dass eine derartige Trochanterstütze an der Tragplatte in Entfall kommen kann, ohne dass dies die Stabilität eines in den Knochen eingewachsenen Prothesenkörpers nachteilig beeinflusst. So kann bei einem erfindungsgemäßen Prothesenkörper gegenüber einem im Stand der Technik bekannten Prothesenkörper eine derartige Trochanterstütze entfallen.

Beispielsweise kann hierfür der Prothesenkörper auf der Höhe der Tragplatte bzw. die Tragplatte selbst lateral zum Schaft hin abfallend enden. Tragplatten mit einer Trochanterstütze hingegen endeten lateral in der Höhe ansteigend, um mit diesem ansteigenden Bereich gegen den Corticalisknochen des Trochanter abgestützt werden zu können. Es ergibt sich somit bei der erfindungsgemäßen Ausgestaltung eine Form des Prothesenkörper, die im oberen Bereich weniger ausladend ist und daher auch einfacher implantierbar ist.

Ebenso kann es hier vorgesehen sein, die Abmaße eines Prothesenkörpers auf der Höhe der Tragplatte derart auszuführen, dass der Prothesenkörper bzw. die Tragplatte selbst parallel zur Längsachse eines Prothesenkopfes bzw. dessen Halses, der an der Tragplatte angeordnet ist, in einem Abstand von 20 bis 30 mm, bevorzugt 22 bis 26 mm und besonders bevorzugt von 24 mm endet. Bei diesen geringen Abständen wird sichergestellt, dass lateral an dem Prothesenkörper kein Element oder Bereich angeordnet ist, der sich abstützend gegen den Trochanter major bzw. dessen Corticalisknochen nach der Insertion eines erfindungsgemäßen Prothesenkörper anlegt.

Bei den hier beschriebenen erfindungsgemäßen Prothesenkörpern kann es vorgesehen sein, dass auf der Tragplatte ein Prothesenkopf fest angeordnet ist. Dieser Prothesenkopf weist einen Halsbereich auf, der in den oberen Bereich der Tragplatte übergeht, wobei an dem Prothesenkopf eine Gelenkkugel befestigt ist oder befestigbar sein kann.

In einer alternativen und besonders bevorzugten Ausführungsform kann es vorgesehen sein, dass ein Prothesenkopf mit seinem Halsbereich an einer Tragplatte befestigbar ist. An einem erfindungsgemäßen Prothesenkörper kann somit nachträglich beispielsweise auch erst nach einer Insertion des Prothesenkörpers in den Femur ein Prothesenkopf befestigt werden.

Hierbei kann es erfindungsgemäß weiterhin vorgesehen sein, dass ein Prothesenkopf in verschiedenen Winkelstellungen an einer Tragplatte befestigbar ist, beispielsweise in wenigstens zwei Winkelstellungen. Es kann sich hierbei bevorzugt um diskrete Winkelstellungen handeln, d.h. ein Prothesenkopf kann in der einen oder anderen Winkelstellung befestigt werden, nicht jedoch in Zwischenpositionen.

Beispielsweise kann es hier vorgesehen sein, dass der Halsbereich oder ein daran angeordneter Vorsprung bzw. Befestigungsteil in ein Befestigungsgegenstück, z.B. eine Ausnehmung, insbesondere eine von wenigstens zwei Ausnehmungen an der Tragplatte einsteckbar ist. Solche Ausnehmungen, beispielsweise zwei Ausnehmungen, können sich zumindest in ihren oberen Bereichen überschneiden.

Beispielsweise können solche Ausnehmungen jeweils als Bohrung ausgebildet sein, wobei sich die Bohrungen ausgehend von einer gemeinsamen Öffnung in der Tragplatte unterhalb der Tragplatte in zwei oder je nach Anzahl der Bohrungen auch mehr verschiedene Richtungen erstrecken. Ein Prothesenkopf, der mit seinem Halsbereich durch eine derartige gemeinsame Öffnung gesteckt wird, kann somit je nach Steckrichtung in eine der vorgesehenen Bohrungen eingesetzt werden und somit verschiedene Winkel in derselben Tragplatte einnehmen.
Bevorzugt ist es hier vorgesehen, bei einer Tragplatte der eingangs genannten erfindungsgemäßen Art, die nach einer Insertion horizontal ausgerichtet ist und insbesondere senkrecht zur Achse der Längserstreckung des Schaftes angeordnet ist, den Hals des Prothesenkopfes in einem Winkel von 58 Grad zur Prothesentragplatte anzuordnen, d.h. in der so genannten Valgusstellung. Hierdurch werden Überbelastungen zwischen Knochen und Implantat insbesondere am Adam'schen Bogen vermieden. In einer anderen Ausführung kann es ebenso vorgesehen sein, den Prothesenkopfhals in einem Winkel von 30 Grad zur Tragplatte anzuordnen, je nachdem welche Situation am Patienten vorgefunden wird.

Es ist daher besonders bevorzug vorgesehen, dass bei befestigbaren Prothesenköpfen je nach Einsteckrichtung einer dieser beiden zuvor genannten Winkel erzielt wird. Dabei ist es erfindungsgemäß vorgesehen, dass die vorgenannten Winkel nicht nur mit an der Tragplatte befestigbaren Prothesenköpfen erzielt wird, sondern ebenso auch mit Prothesenköpfen, die fest bereits bei der Herstellung mit einer Tragplatte verbunden sind. Es kann daher im Wesentlichen vorgesehen sein, zwei verschiedene Prothesenkörper bereitzustellen, einen mit einem Winkel von 58 Grad und einen mit einem Winkel von 30 Grad zwischen Prothesenhals und Tragplatte. Selbstverständlich sind hier andere Winkel möglich, sofern diese sich als geeignet erweisen.

In einer weiterhin bevorzugten Ausführungsform, die alleinig oder auch in Verbindung mit den vorgenannten Ausführungen einsetzbar ist, ist es vorgesehen, dass der Prothesenkörper wenigstens ein Verbindungselement, insbesondere jeweils ein Verbindungselement in einem proximalen und einem distalen Bereich aufweist, welches beispielsweise als Gewindebohrung ausgeführt sein kann und mit dem der Prothesenkörper mit einer Vibrationsvorrichtung verbindbar ist. Beispielsweise kann ein solches Verbindungselement, insbesondere eine genannte Gewindebohrung in der Tragplatte und/oder am unteren Schaftende (Olive) ventral und/oder dorsal angeordnet sein.

Durch die Ankopplung einer Vibrationsvorrichtung an ein solches Verbindungselement, insbesondere an zwei Stellen des Prothesenkörpers, kann eine Prothese, die in einen Knochen eingewachsen ist, nachträglich wieder aus dem Knochen durch die Applikation von Oszillationen an die Prothese entfernt werden. So können mittels einer Vibrationsvorrichtung, beispielsweise über Stäbe, die in die Gewindebohrungen im dorsalen und proximalen Bereich eingeschraubt werden, Vibrationen in den Prothesenkörper eingeleitet werden, wodurch kontrolliert eine Zertrümmerung des Spongiosa-Knochens um den Prothesenkörper bzw. dessen Schaft herum erzielt werden kann. Es besteht so die Möglichkeit, nach einer Lockerung des Prothesenkörpers im Spongiosa-Knochen diesen wiederum aus dem Femur herauszuziehen, beispielsweise mit einem einzigen gezielt angesetzten Schlag.

Um die Gewindebohrungen im Prothesenkörper zugänglich zu machen, insbesondere eine dorsal angeordnete Gewindebohrung am unteren Schaftende, ist es vorgesehen, Zugänge durch den Knochen zu erstellen, um sodann eine Vibrationsvorrichtung anschließen zu können.

In einer weiterhin bevorzugten Ausführung kann es vorgesehen sein, dass der Hals eines Prothesenkopfes relativ zur ventralen Stirnfläche der Tragplatte in einem Winkel von 4 - 5 Grad, insbesondere in einem Winkel von 4,4 Grad angeordnet ist. Auch hierbei ergeben sich physiologische Vorteile einer derartigen erfindungsgemäßen Prothese.

In einer weiteren erfindungsgemäßen Ausgestaltung kann es auch vorgesehen sein, in den Prothesenkörper einen Chip einzusetzen, der von außen berührungslos auslesbar ist. Bei einem solchen Chip kann es sich z.B. um einen RFID Chip handeln. Dies hat den Vorteil, dass zum einen der verwendete Prothesenkörper identifiziert werden kann, z.B. durch eine gespeicherte Seriennummer und dass zum anderen auch z.B. relativ zu einer festen Ursprungsposition in dem Chip eine Koordinate abgespeichert sein kann, die von außen auslesbar ist und die angibt, wo relativ zu der Ursprungsposition ein Verbindungselement angeordnet ist, um eine Vibrationsvorrichtung an dem Prothesenkörper befestigen zu können. So kann durch das Auslesen dieser Koordinateninformation ein Operateur die Lage identifizieren, in der z.B. eine Bohrung in den Knochen eingebracht werden muss, die an einem Verbindungselement, insbesondere einer Gewindebohrung endet, um sodann ein die Vibration leitenden Stab der Vibrationsvorrichtung hier anbringen zu können.

Ein beispielhaftes eingangs genanntes Raspelinstrument für einen Prothesenkörper der vorgenannten erfindungsgemäßen Art, welches auf seiner Oberfläche Raspelvorsprünge aufweist, um die Spongiosa im proximalen Ende eine Femurs auszuraspeln, zeichnet sich dadurch aus, dass es zum Einen mit seiner Oberfläche formgleich ist zu der Oberfläche des Prothesenkörpers, zumindest zu der Oberfläche einer gedachten Hülle des Prothesenkörpers, die gedacht um die Schaftpfosten herumgelegt ist. Hierbei ist es bei einer Ausführungsform eines Raspelinstruments dieser Art vorgesehen, dass die Oberfläche des Prothesenkörpers größer ist als die des Raspelinstrumentes, insbesondere derart, dass der Querschnitt des Prothesenkörpers an jeder Stelle 0,5 - 4 mm, bevorzugt 1 - 3 mm und besonders bevorzugt 2 mm größer ist. Hierdurch kann beim Einsetzen des Prothesenschaftes in das Spongiosavolumen, welches mit dem kleineren Raspelinstrument erzeugt wurde, ein ausreichender Presssitz erzeugt werden zwischen Prothesenschaft und den inneren Wandbereichen des Corticalisknochens und dem Rest des Songiosaknochens, so dass sich eine Form- und Sitzstabilität bis zu vollständigen Einheilung der Spongiosa im implantierten Prothesenkörper ergibt.

Ausführungsbeispiele der Erfindung sind in den nachfolgend beschriebenen Figuren dargestellt. Es zeigen:
- Fig. 1:: eine schematische Darstellung eines erfindungsgemäßen Prothesenkörpers nach Insertion in einen Oberschenkelknochen;
- Fig. 2:: in mehreren Ansichten einen erfindungsgemäßen Prothesenkörper;
- Fig. 3:: die Aufsicht auf einen erfindungsgemäßen Prothesenkörper im Vergleich zum Prothesenkörper gemäß Stand der Technik.

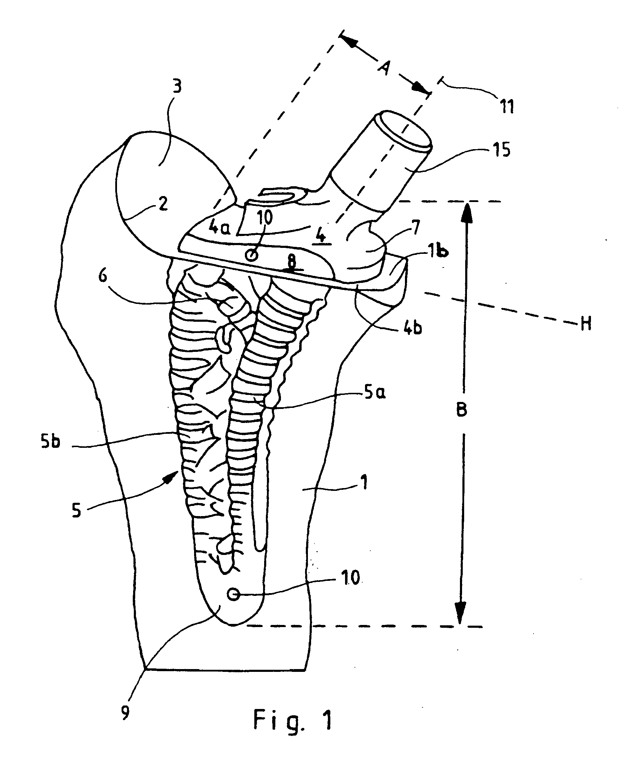

In der Fig. 1 ist ein Prothesenkörper der erfindungsgemäßen Art dargestellt, der in den oberen proximalen Teil eines Oberschenkelknochens 1 eingesetzt ist. Hierfür wurde eine Resektion des proximalen Teils vorgenommen entlang der Resektionslinie 2, wobei ein signifikanter Teil des Trochanter major 3 erhalten geblieben ist.

Erkennbar ist hier, dass der Prothesenkörper eine Tragplatte 4 aufweist, von der ausgehend nach unten zwei Schaftpfosten 5a und 5b herabhängen, die am unteren Verbindungsbereich der Tragplatte 4 mit dieser fest verbunden sind. Beispielsweise können der obere Teil des Prothesenkörpers mit der Tragplatte 4 und der untere mit den Schaftpfosten 5a und 5b gemeinsamen in einem Gießprozess hergestellt worden sein. Die hinteren zwei Schaftpfosten sind projektionsbedingt nicht darstellbar.

Es ist hier weiterhin erkennbar, dass sich zwischen den Schaftpfosten 5a und 5b schräge Verstrebungen 6 erstrecken, um die Schaftpfosten 5a und 5b miteinander zu verbinden und den gesamten Schaft 5 zu stabilisieren. Dabei ergeben sich zwischen den Schaftpfosten 5a und 5b sowie den Verstrebungen 6 Ausnehmungen sowie offene Durchgangsbereiche 12, in die nach der Insertion eines derartigen Prothesenkörpers Spongiosa einwachsen kann. Bevorzugt soll das Metallvolumen des Prothesenschaftes das Spongiosavolumen, in welchem der Prothesenschaft sitzt, nicht mehr als 9-12% überschreiten.

Hier ist weiterhin erkennbar, dass bei der erfindungsgemäßen Ausführung dieses Prothesenkörpers die Tragplatte 4 lediglich einen medialen Überstand bzw. Kragenbereich 7 aufweist, der über den medialen Schaftpfosten 5a bzw. den Querschnitt des proximalen Endes des Schaftes direkt unterhalb der Tragplatte 4 übersteht, insbesondere so, dass sich dieser überstehende Bereich 7 mit seiner Unterfläche 4b auf den Corticalisknochen 1b des Femurs 1 auflegt, der an dieser Stelle besonders stabil ist.

Weiterhin zeigt die Darstellung, dass die ventrale Seitenfläche 8 der Tragplatte 4 ebenso wie die hier nicht sichtbare dorsale Seitenfläche der Tragplatte 4 keinen oder zumindest nur einen im Sinne der Erfindung unwesentlichen Überstand gegenüber dem Schaft direkt unterhalb der Tragplatte 4 aufweist. Hierdurch ergibt sich, dass die Tragplatte im ventralen und dorsalen Bereich nicht auf dem Corticalisknochen des Femurs aufliegt, sondern medial im Femur 1 angeordnet ist.

Es zeigt sich darüber hinaus weiterhin, dass das laterale Ende 4a der Tragplatte 4 keinen nach oben aufstehenden Bereich ausbildet wie er im Stand der Technik bekannt ist, sondern die Tragplatte 4 hier im Wesentlichen in Verlängerung des darunter liegenden Schaftes 5 endet. Hier kann die Tragplatte einen zum Schaft 5 nach unten hin abfallenden Bereich aufweisen. Insbesondere ist es jedoch in jedem Fall erfindungsgemäß vorgesehen, dass das laterale Ende 4a der Tragplatte bzw. der Prothesenkörper in seinem lateralen Bereich derart endet, dass dieser nach der Insertion zumindest im Wesentlichen anlagefrei zum Trochanter major 3 ausgebildet ist, sich also dort zumindest nicht wesentlich abstützt.

Bei der hier dargestellten Ausführung des erfindungsgemäßen Prothesenkörpers ist erkennbar, dass an der Tragplatte 4 fest ein Prothesenkopf 15 befestigt ist, dessen Längsachse zur Horizontalen H, in welcher zumindest die Unterseite der Tragplatte 4 angeordnet ist, einen Winkel von 58 Grad einnimmt. Ebenso wäre hier ein Winkel von 30 Grad bevorzugt vorgesehen.

An dem Prothesenkopf 15 ist hier in bekannter Weise eine Gelenkkugel befestigbar, die zu einer Gelenkpfanne im Hüftknochen korrespondiert.

Die hier dargestellte Ausführungsform zeigt eine weitere bevorzugte Weiterbildung, die vorgesehen sein kann, jedoch nicht zwingend vorgesehen sein muss und die darin besteht, dass sowohl am distalen Ende 9 des Prothesenschaftes 5 wie auch an dessen proximalen Ende und hier insbesondere in der ventralen Seitenfläche 8 der Tragplatte 4 eine Gewindebohrung 10 angeordnet ist. In eine solche Gewindebohrung 10 kann nach Schaffung eines entsprechenden Zugangs durch den Knochen hindurch ein Stab eingeschraubt werden, mittels dem Vibrationen von einer Vibrationsvorrichtung in den Prothesenkörper eingeleitet werden können, um diese innerhalb des Spongiosaknochens ebenso zu einer Vibration anzuregen und hierdurch den Spongiosaknochen um den Schaft 5 herum zu zertrümmern, so dass nach einem Einheilen eines solchen Prothesenkörpers dieser auch wieder aus dem Oberschenkelknochen nachträglich entfernt werden kann.

Die hier dargestellte erfindungsgemäße Ausführung zeigt weiterhin, dass das laterale Ende der Tragplatte 4 im Bereich 4a einen Abstand parallel zur Längsachse 11 des Prothesenkopfes 15 aufweist, der hier bevorzugt zu A = 20 - 30 mm, weiterhin bevorzugt 22 - 26 mm und besonders bevorzugt 24 mm gewählt ist. Auch hierdurch wird sichergestellt, dass sich nach der Insertion des Prothesenkörpers im Wesentlichen keine Anlage des Prothesenkörpers bzw. dessen Tragplatte 4 zum Trochanter major ergibt.

Das Maß B zwischen distalem Ende des Prothesenkörpers und einem Werkzeugansatz kann hier bevorzugt zu B=104 mm gewählt sein. Ebenso sind andere Längen möglich.

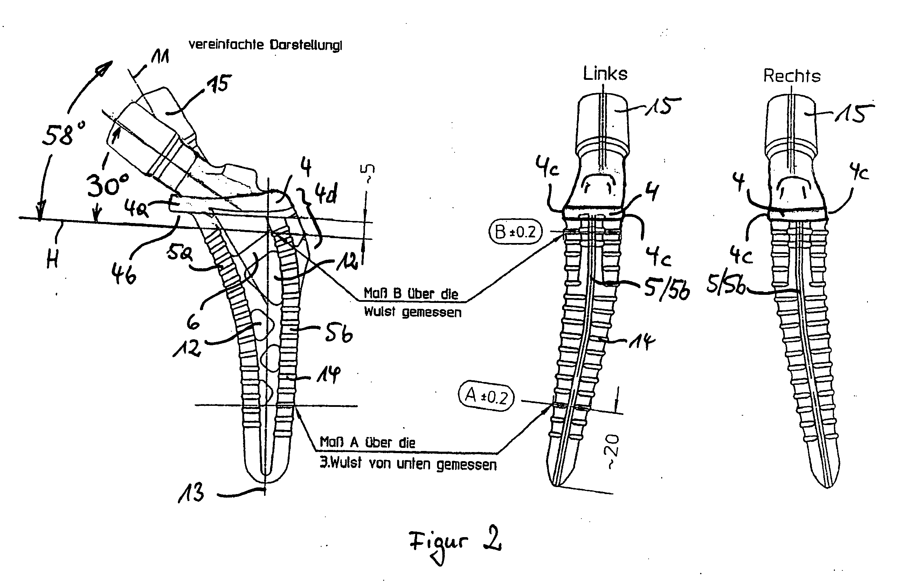

Die Fig. 2 zeigt in mehreren Ansichten erfindungsgemäße Prothesenkörper, wobei hier erkennbar ist, dass die Tragplatte 4 lediglich in ihrem medialen Bereich 4a, der dem Hüftknochen zugewandt ist, einen Vorsprung gegenüber dem Schaft und hier insbesondere dem medialen Schaftpfosten 5a aufweist, so dass sich die Unterseite 4b dieses medialen Kragens 4a auf den Corticalisknochen des Oberschenkels auflegen kann. Die mit "links" bzw. "rechts" überschriebenen rechtsseitigen Darstellungen des erfindungsgemäßen Prothesenkörpers für einen linken bzw. rechten Oberschenkelknochen zeigen bei einer Ansicht von lateral, dass die ventralen und dorsalen Seitenflächen 4c nicht bzw. nur unwesentlich in einem Bereich von weniger als 10 % gegenüber dem Schaft 5 direkt unterhalb der Tragplatte 4 überstehen.

Erkennbar ist bei der hier gewählten Darstellung ebenso, dass zwischen dem medialen Schaftpfosten 5a und dem lateralen Schaftpfosten 5b sich Schrägverstrebungen 6 erstrecken und weiterhin in den Zwischenbereichen Ausnehmungen bzw. freie Durchgänge 12 gegeben sind, in die Spongiosamaterial einwachsen kann.

Erkennbar ist hier in der Fig. 2 weiterhin eine Ausführung, bei der ein Prothesenkopf 15 in verschiedenen Winkelstellungen in die Tragplatte 4 eingesteckt werden kann. Hier sind in der Zeichnung übereinander gelagert dargestellt zwei Winkelstellungen gezeigt, bei denen der Prothesenkopf 15 entweder in einem Winkel von 30 Grad oder in einem Winkel von 58 Grad zur Horizontalen H, d.h. der Ausrichtung zumindest der Unterseite der Tragplatte 4 angeordnet ist. Dabei ist die Tragplatte 4 mit ihrer Unterseite wiederum senkrecht ausgerichtet zur hier vertikal dargestellten Erstreckungsachse 13 des Schaftes 5.

Deutlich zeigt auch die Fig. 2 hier insbesondere in der linksseitigen Darstellung, dass die Tragplatte 4 lateral, d.h. oberhalb des Schaftpfostens 5b nach unten abfallend hier insbesondere in dem dargestellten Bereich 4d ausgebildet ist. Diese hier dargestellte schräge Seitenfläche 4d weist insbesondere zu der Mittenachse 11 des Prothesenkopfes 15 unter einem Winkel von 58 Grad einen parallelen Abstand auf, der bevorzugt in dem eingangs genannten Bereich liegt von 20 -30 mm, bevorzugt 22 - 26 mm und besonders bevorzugt 24 mm.

Wesentlich ist jedoch auch hier festzustellen, dass die Tragplatte 4 lateral keinen nach oben vorstehenden und dabei gleichzeitig über die Verlängerung des Schaftpfostens 5b lateral überstehenden Trochanter Stützbereich aufweist.

Die Figuren 2 zeigen hier ebenso wie auch schon die Fig. 1, dass in axialer Erstreckung der Schaftpfosten 5a und 5b im jeweiligen Abstand zueinander Verdickungen, so genannte Verankerungsbunde 14 vorgesehen sind, die ebenfalls zu einer erhöhten Stabilität nach dem Einwachsen in den Oberschenkelknochen dienen. Die wichtigste Funktion dieser Verankerungsbunde ist die Oberflächenvergrößerung.

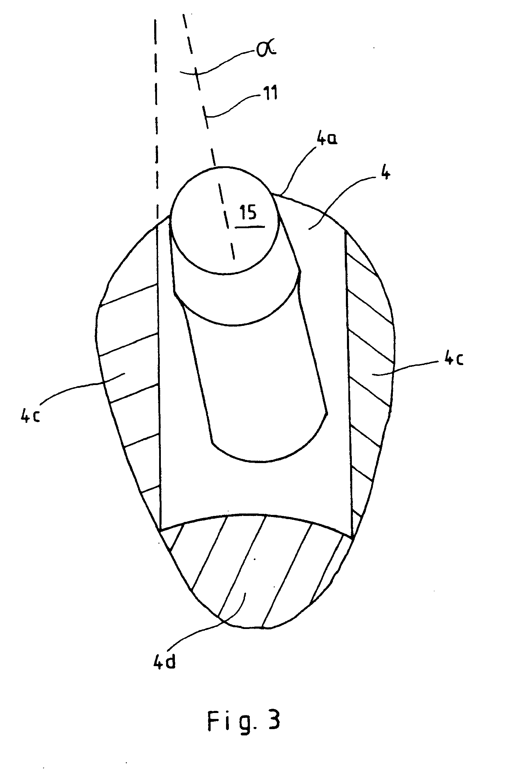

Die Fig. 3 zeigt einen direkten Vergleich in Aufsicht von oben auf einen erfindungsgemäßen Prothesenkörper gegenüber einem Prothesenkörper wie er im Stand der Technik bekannt ist. Die hier schraffiert dargestellten Flächenbereiche 4c, 4d zeigen die äußeren Abmessungen einer Tragplatte 4 gemäß eines Prothesenkörpers nach dem Stand der Technik bei sehr proximaler Resektion.
Die Resektion verläuft horizontal, liegt aber im proximalen Schenkelhalsbereich sowie teilweise im distalen Bereich des Femurkopfes.

Es ist hier erkennbar, dass sich ein erfindungsgemäßer Prothesenkörper gegenüber dem bekannten dadurch unterscheidet, dass von der bekannten Tragplatte 4 ventrale und dorsale Seitenbereiche 4c soweit entfallen, dass sich ventrale und dorsale Seitenflächen der Tragplatte 4 ergeben, die nicht oder zumindest nur unwesentlich im Sinne der Erfindung über den darunter liegenden Schaft überstehen. Ebenso ist hier auch ein lateraler Bereich 4d in Entfall gekommen, so dass die Tragplatte 4 lediglich im medialen Bereich 4a über den darunter liegenden Schaft übersteht. In Betrachtung von oben hingegen sind die ventralen, dorsalen und lateralen Begrenzungen der Tragplatte mit dem darunter liegenden Schaft entweder fluchtend, insbesondere hinsichtlich ventralem und dorsalem Bereich, allenfalls unwesentlich überstehend oder insbesondere hinsichtlich des lateralen Bereiches sogar zurückstehend.

Die Fig. 3 zeigt hier weiterhin auch, dass es erfindungsgemäß vorgesehen ist in einer Ausführung, dass die Längsachse 11 des Prothesenkopfes 15 gegenüber der ventralen Seitenfläche der Tragplatte 4 einen Winkel α einnimmt, der hier bevorzugt in einem Bereich von 4 - 5 Grad und besonders bevorzugt von 4,4 Grad gewählt sein kann.

Bezüglich sämtlicher Ausführungen ist festzustellen, dass die in Verbindung mit einer Ausführung genannten technischen Merkmale nicht nur bei der spezifischen Ausführung eingesetzt werden können oder eingesetzt sind, sondern auch bei den jeweils anderen Ausführungen. Sämtliche offenbarten technischen Merkmale dieser Erfindungsbeschreibung sind als erfindungswesentlich einzustufen und beliebig miteinander kombinierbar oder in Alleinstellung einsetzbar. Dabei wird in der gesamten Offenbarung unter der Erwähnung, dass ein Merkmal vorgesehen sein kann oder ein Verfahrenschritt durchgeführt werden kann auch eine Ausführung der Erfindung verstanden, in der das betreffende Merkmal vorgesehen ist bzw. ein betreffender Verfahrensschritt durchgeführt wird.

## Patentansprüche

1. Prothesenkörper für eine Oberschenkelprothese umfassend einen Schaft (5, 5a, 5b) mit einer proximalen Tragplatte (4) für einen Prothesenkopf (15), wobei an der Tragplatte (4) von dieser herabhängend wenigstens zwei proximal beabstandete und distal aufeinander zulaufende Schaftpfosten (5a, 5b) befestigt sind, die durch Verstrebungen (6) verbunden sind und wobei zwischen den Schaftpfosten (5a, 5b) und den Verstrebungen (7) Ausnehmungen (12) und/oder frei durchgängige Bereiche (12) gebildet sind, in die Spongiosa einwachsen kann, **dadurch gekennzeichnet, dass**
a. zumindest der untere Verbindungsbereich der Tragplatte (4), an dem die Schaftpfosten (5a, 5b) befestigt sind, horizontal angeordnet ist,
b. die Tragplatte (4) medial über den unmittelbar unterhalb des Verbindungsbereiches gegebenen Querschnitt des Schaftes (5, 5a, 5b) übersteht und
c. die Tragplatte (4) ventral und dorsal ohne Überstand in den Schaft (5, 5a, 5b) übergeht.

2. Prothesenkörper nach Anspruch 1, **dadurch gekennzeichnet, dass** der Prothesenkörper lateral im Bereich der Tragplatte (4) derart endet, dass er nach einer Insertion in den Femur (1) zumindest überwiegend anlagefrei zur Corticalis des Trochanter major (3) ist.

3. Prothesenkörper nach einem der vorherigen Ansprüche, **dadurch gekennzeichnet, dass** der Prothesenkörper auf der Höhe der Tragplatte (4) lateral zum Schaft hin abfallend endet.

4. Prothesenkörper nach einem der vorherigen Ansprüche, **dadurch gekennzeichnet, dass** der Prothesenkörper auf der Höhe der Tragplatte (4) in einem Abstand parallel zur Längsachse (11) des Prothesenkopfes (15) oder dessen Hals von 20 bis 30 mm, bevorzugt 22 bis 26 mm, besonders bevorzugt 24 mm endet.

5. Prothesenkörper nach einem der vorherigen Ansprüche, **dadurch gekennzeichnet, dass** ein Prothesenkopf (15) mit seinem Halsbereich in wenigstens zwei verschiedenen Winkelstellungen, an der Tragplatte (4) oder einem daran angeordneten Aufnahmebereich befestigbar ist.

6. Prothesenkörper nach Anspruch 5, **dadurch gekennzeichnet, dass** der Halsbereich oder ein daran angeordneter Vorsprung in eine von wenigstens zwei Ausnehmungen an der Tragplatte (4) einsteckbar ist.

7. Prothesenkörper nach Anspruch 6, **dadurch gekennzeichnet, dass** zwei Ausnehmungen sich zumindest in ihren oberen Bereichen zumindest teilweise überschneiden.

8. Prothesenkörper nach einem der vorherigen Ansprüche, **dadurch gekennzeichnet, dass** er wenigstens ein Verbindungselement (10) aufweist, mit dem der Prothesenkörper mit einer Vibrationsvorrichtung verbindbar ist.

9. Prothesenkörper nach Anspruch 8, **dadurch gekennzeichnet, dass** ein Verbindungselement (10) in der Tragplatte (4) und/oder am unteren Schaftende (9) ventral und/oder dorsal angeordnet ist.

10. Prothesenkörper nach einem der vorherigen Ansprüche, **dadurch gekennzeichnet, dass** der Hals eines Prothesenkopfes (15) relativ zur ventralen Stirnfläche der Tragplatte in einem Winkel von 4 - 5 Grad angeordnet ist.

11. Prothesenkörper nach einem der vorherigen Ansprüche, **dadurch gekennzeichnet, dass** an/in ihm ein Chip zur Speicherung und Auslesung von Daten angeordnet ist.

## Claims

1. Prosthestic body for an upper leg prosthesis, comprising a shaft (5, 5a, 5b) with a proximal support plate (4) for a prosthesis head (15), wherein at least two shaft posts (5a, 5b), which are connected by struts (6), are secured on and hang down from the support plate (4), said shaft posts (5a, 5b) being spaced apart at the proximal end and tapering towards each other at the distal end, and wherein recesses (12) and/or free open areas (12), into which spongy bone can grow, are formed between the shaft posts (5a, 5b) and the struts (6), **characterized in that**
a. at least the lower connection area of the support plate (4), on which the shaft posts (5a, 5b) are secured, is arranged horizontally,
b. the support plate (4) protrudes medially beyond the cross section of the shaft (5, 5a, 5b) provided directly below the connection area, and
c. the support plate (4) merges ventrally and dorsally into the shaft (5, 5a, 5b) without protrusion.

2. Prosthetic body according to Claim 1, **characterized in that** the prosthetic body ends laterally in the area of the support plate (4) in such a way that, after insertion into the femur (1), the prosthetic body is at least largely free of contact with the cortical bone of the greater trochanter (3).

3. Prosthetic body according to either of the preceding claims, **characterized in that** the prosthetic body ends, at the level of the support plate (4), falling away laterally towards the shaft.

4. Prosthetic body according to one of the preceding claims, **characterized in that** the prosthetic body, at the level of the support plate (4), ends at a distance, parallel to the longitudinal axis (11) of the prosthesis head (15) or the neck thereof, of 20 to 30 mm, preferably 22 to 26 mm, particularly preferably 24 mm.

5. Prosthetic body according to one of the preceding claims, **characterized in that** a prosthesis head (15) can be secured with its neck area in at least two different angle positions on the support plate (4) or on a receiving area arranged thereon.

6. Prosthetic body according to Claim 5, **characterized in that** the neck area, or a projection arranged thereon, can be plugged into one of at least two recesses on the support plate (4).

7. Prosthetic body according to Claim 6, **characterized in that** two recesses at least partially intersect each other, at least in their upper areas.

8. Prosthetic body according to one of the preceding claims, **characterized in that** it has at least one connection element (10), with which the prosthetic body can be connected to a vibration device.

9. Prosthetic body according to Claim 8, **characterized in that** a connection element (10) is arranged ventrally and/or dorsally in the support plate (4) and/or at the lower shaft end (9).

10. Prosthetic body according to one of the preceding claims, **characterized in that** the neck of a prosthesis head (15) is arranged at an angle of 4-5 degrees relative to the ventral end face of the support plate.

11. Prosthetic body according to one of the preceding claims, **characterized in that** a chip for storing and reading out data is arranged thereon/therein.

## Revendications

1. Corps prothétique pour une prothèse fémorale, comprenant une tige (5, 5a, 5b) avec une plaque support proximale (4) pour une tête prothétique (15), au moins deux colonnes de tige (5a, 5b) espacées au niveau de la partie proximale et convergeant l'une vers l'autre au niveau de la partie distale étant fixées à la plaque support (4) de manière suspendue à celle-ci, lesquelles sont connectées par des membrures (6), et des évidements (12) et/ou des régions de passage libre (12) étant formé(e)s entre les colonnes de tige (5a, 5b) et les membrures (6), dans lesquel(le)s peut croître du tissu spongieux, **caractérisé en ce que**
a. au moins la région de liaison inférieure de la plaque support (4) à laquelle sont fixées les colonnes de tige (5a, 5b) est disposée horizontalement,
b. la plaque support (4) se prolonge dans une direction médiale au-delà de la section transversale de la tige (5, 5a, 5b) située directement en dessous de la région de liaison, et
c. la plaque support (4) se prolonge sans dépassement dans la tige (5, 5a, 5b) dans la direction ventrale et dorsale.

2. Corps prothétique selon la revendication 1, **caractérisé en ce que** le corps prothétique se termine dans la direction latérale dans la région de la plaque support (4) de telle sorte qu'après une insertion dans le fémur (1), il soit au moins largement sans contact d'appui avec la corticale du grand trochanter (3).

3. Corps prothétique selon l'une quelconque des revendications précédentes, **caractérisé en ce que** le corps prothétique se termine à la hauteur de la plaque support (4) en descendant latéralement vers la tige.

4. Corps prothétique selon l'une quelconque des revendications précédentes, **caractérisé en ce que** le corps prothétique se termine à la hauteur de la plaque support (4) à une distance parallèle à l'axe longitudinal (11) de la tête prothétique (15) ou de son col de 20 à 30 mm, de préférence de 22 à 26 mm, particulièrement préférablement de 24 mm.

5. Corps prothétique selon l'une quelconque des revendications précédentes, **caractérisé en ce qu'**une tête prothétique (15) peut être fixée par sa région de col dans au moins deux positions angulaires différentes, à la plaque support (4) ou à une région de réception disposée sur celle-ci.

6. Corps prothétique selon la revendication 5, **caractérisé en ce que** la région de col ou une saillie disposée sur celle-ci peut être enfichée dans l'un d'au moins deux évidements sur la plaque support (4).

7. Corps prothétique selon la revendication 6, **caractérisé en ce que** deux évidements se recoupent au moins en partie au moins dans leurs régions supérieures.

8. Corps prothétique selon l'une quelconque des revendications précédentes, **caractérisé en ce qu'**il présente au moins un élément de liaison (10) avec lequel le corps prothétique peut être relié à un dispositif vibrateur.

9. Corps prothétique selon la revendication 8, **caractérisé en ce qu'**un élément de liaison (10) est disposé dans la plaque support (4) et/ou au niveau de l'extrémité inférieure de la tige (9) en position ventrale et/ou dorsale.

10. Corps prothétique selon l'une quelconque des revendications précédentes, **caractérisé en ce que** le col d'une tête prothétique (15) est disposé par rapport à la surface frontale ventrale de la plaque support suivant un angle de 4 à 5 degrés.

11. Corps prothétique selon l'une quelconque des revendications précédentes, **caractérisé en ce qu'**une puce pour mémoriser et lire des données est disposée sur/dans celui-ci.
